# EUROPÄISCHE PATENTANMELDUNG

(11) **EP 3 473 323 A1**
(43) Veröffentlichungstag der Anmeldung: **24.04.2019**
(21) Anmeldenummer: 17197382.9
(22) Anmeldetag: 19.10.2017
(51) Int. Cl.: B01D 53/047

(54) **PROZESS UND ANLAGE ZUR GEWINNUNG VON ETHYLEN**

(71) Anmelder: Linde AG, 80331 München (DE)
(72) Erfinder: Leitmayr, Werner, 86633 Neuburg/Donau (DE); Mündl, Florian, 83624 Otterfing (DE); Ernst, Christian, 85521 Ottobrunn (DE)
(74) Vertreter: m patent group

(57) **Zusammenfassung**

Die Erfindung betrifft einen Prozess (100) zur Herstellung von Ethylen, bei dem unter Einsatz eines Verfahrens zur oxidativen Kopplung von Methan (10) ein erstes Gasgemisch (1) gebildet wird, bei dem ein Teil des ersten Gasgemischs (1) zur Bildung eines zweiten Gasgemischs (2) verwendet wird, und bei dem zumindest ein Teil des zweiten Gasgemischs (2) einer Vakuumdruckwechseladsorption (50) unterworfen und zur Bildung eines dritten Gasgemischs (3) und eines vierten Gasgemischs (4) verwendet wird, wobei das zweite Gasgemisch (2) Methan, Ethan und Ethylen enthält, wobei das dritte Gasgemisch (3) gegenüber dem zweiten Gasgemisch (5) an Methan angereichert und an Ethan und Ethylen abgereichert ist, und wobei das vierte Gasgemisch (4) gegenüber dem zweiten Gasgemisch (5) an Methan abgereichert und an Ethan und Ethylen angereichert ist. Es ist vorgesehen, dass ein erster Anteil des vierten Gasgemischs (4) nach der Bildung des ersten Gasgemischs (1) mit dem ersten Gasgemisch (1) oder mit dessen zur Bildung des zweiten Gasgemischs (2) verwendetem Teil vereinigt und zur Bildung des zweiten Gasgemischs (2) verwendet wird, und dass ein zweiter Anteil des vierten Gasgemischs anderweitig verwendet wird. Eine entsprechende Anlage ist ebenfalls Gegenstand der vorliegenden Erfindung.

## Beschreibung

Die Erfindung betrifft einen Prozess zur Gewinnung von Ethylen sowie optional weiterer Olefine und eine entsprechende Anlage gemäß den Oberbegriffen der jeweiligen unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet. Der eingesetzte Sauerstoff wird bei den genannten Reaktionen typischerweise vollständig umgesetzt.

Die Reaktionsbedingungen bei der oxidativen Kopplung von Methan umfassen klassischerweise eine Temperatur von 500 bis 900 °C, einen Druck von 5 bis 10 bar und hohe Raumgeschwindigkeiten. Jüngere Entwicklungen gehen insbesondere auch in Richtung der Verwendung tieferer Temperaturen. Die Reaktion kann homogen- und heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden, der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Typischerweise werden nicht mehr als 10 bis 15% des eingesetzten Methans umgesetzt. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid.

In Prozessen und Anlagen zur oxidativen Kopplung von Methan werden daher zunächst Gasgemische erhalten, die neben primären Zielverbindungen wie Ethan und Ethylen überwiegend nicht umgesetztes Methan sowie Kohlendioxid, Kohlenmonoxid, Wasser und weitere Nebenprodukte enthalten. Durch parallel zu der oxidativen Kopplung von Methan ablaufende nichtkatalytische Spaltreaktionen können auch beträchtliche Mengen an Wasserstoff gebildet werden.

Bei der oxidativen Kopplung von Methan können Reaktoren mit mehreren Reaktionszonen oder entsprechende Reaktoranordnungen mit mehreren Reaktoren, die jeweils eine oder mehrere entsprechende Reaktionszonen aufweisen, eingesetzt werden. Insbesondere kann ein durch die eigentliche oxidative Kopplung von Methan gebildetes Gasgemisch der soeben erläuterten Art aus einer oder mehreren katalytischen Reaktionszonen zumindest zum Teil in eine oder mehrere nichtkatalytische Reaktionszonen überführt werden.

In der oder den nichtkatalytischen Reaktionszonen liegen zunächst noch vergleichsweise hohe Temperaturen vor. Insbesondere durch die Anwesenheit des bei der oxidativen Kopplung von Methan gebildeten Wassers ähneln daher die Reaktionsbedingungen hier jenen herkömmlicher Dampfspaltverfahren (engl. Steam Cracking). Daher können auf diese Weise Ethan und höhere Paraffine zu Olefinen umgesetzt werden. In eine nichtkatalytische Reaktionszone können auch weitere Paraffine eingespeist werden, so dass die Restwärme der oxidativen Kopplung von Methan in besonders vorteilhafter Weise ausgenutzt werden kann. Ein derartiges gezieltes Dampfspalten wird auch als "Post Bed Cracking" (PBC) bezeichnet. Nachfolgend wird auch der Begriff "postkatalytisches Dampfspalten" verwendet.

Bezüglich den beim Dampfspalten herrschenden Reaktionsbedingungen sei ebenfalls auf Fachliteratur wie den Artikel "Ethylene" in Ullmann's Encyclopedia of Industrial Chemistry, Onlineausgabe, 15. April 2007, DOI 10.1002/14356007.a10_045.pub2, verwiesen. Das Dampfspalten wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Ein in entsprechenden Prozessen dem oder den Reaktoren entnommenes Gasgemisch wird typischerweise einer möglichst raschen Abkühlung, einem sogenannten Quench, unterworfen, um die ablaufenden Reaktionen möglichst rasch zum Stillstand zu bringen und damit eine Weiterreaktion zu unerwünschten Nebenprodukten unterbinden. Dies gilt ungeachtet dessen, ob in entsprechenden Prozessen ein postkatalytisches Dampfspalten erfolgt oder nicht. Zu weiteren Details sei auf die zitierte Fachliteratur zum Thema Dampfspalten verwiesen, da der Zweck des Quenchs hier im Wesentlichen derselbe wie bei der oxidativen Kopplung von Methan ist.

Die sich an den Quench anschließende trenntechnische Bearbeitung des Gasgemischs umfasst herkömmlicherweise eine weitere Kühlung auf Umgebungstemperatur. Hierbei kondensiert der überwiegende Teil des in dem Gasgemisch enthaltenen Wassers aus. Anschließend erfolgen eine Verdichtung und danach eine Entfernung des Kohlendioxids, beispielsweise unter Verwendung einer Amin- und/oder Laugewäsche. Restwasser wird typischerweise adsorptiv, beispielsweise unter Verwendung von Molsiebadsorbern, abgetrennt.

Ein durch eine entsprechende Aufbereitung erhaltenes Gasgemisch kann nach einer Verdichtung einer Tieftemperaturtrennung zugeführt werden, in der beispielsweise Methan und tiefer als Methan siedende Komponenten, also insbesondere Kohlenmonoxid und Wasserstoff, sofern enthalten, zumindest überwiegend abgetrennt werden. Der verbleibende Rest des Gasgemischs enthält dann überwiegend oder ausschließlich Ethylen und höher als Ethylen siedende Verbindungen, soweit in dem Gasgemisch zuvor enthalten.

Die trenntechnische Bearbeitung kann auch insbesondere den Einsatz einer Druckwechseladsorption (engl. Pressure Swing Adsorption, PSA) umfassen. Hierbei kann auch eine Druckwechseladsorption eingesetzt werden, bei der die Desorption bei einem Druckniveau unterhalb des Atmosphärendrucks erfolgt. Diese wird auch als Vakuumdruckwechseladsorption (VPSA) bezeichnet. Durch den Einsatz der Druckwechseladsorption, in der bevorzugt schwerere Komponenten adsorbieren, kann ein entsprechendes Gasgemisch insbesondere an Methan abgereichert werden. Durch die Abreicherung an Methan lassen sich beispielsweise nachfolgende Trennschritte einfacher realisieren, da hier geringere Gasmengen bearbeitet werden müssen. Das Methan kann zur oxidativen Kopplung zurückgeführt werden. Prozesse und Anlagen, in denen eine oxidative Kopplung von Methan und eine Vakuumdruckwechseladsorption eingesetzt werden, sind z.B. aus der DD 275 452 A1, der WO 2015/162090 A1 und der WO 2016/001116 A1 bekannt.

Die vorliegende Erfindung stellt sich die Aufgabe, Prozesse und Anlagen zur Gewinnung von Ethylen und optional weiterer Olefine, in denen zumindest eine oxidative Kopplung von Methan und eine Vakuumdruckwechseladsorption eingesetzt werden, zu verbessern und effizienter zu gestalten.

### Offenbarung der Erfindung

Vor diesem Hintergrund schlägt die vorliegende Erfindung einen Prozess zur Gewinnung von Ethylen und optional weiterer Olefine und eine entsprechende Anlage gemäß den Oberbegriffen der jeweiligen unabhängigen Patentansprüche vor. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden noch einige Grundlagen und die verwendeten Begriffe erläutert.

Ist nachfolgend davon die Rede, dass "unter Einsatz eines Verfahrens zur oxidativen Kopplung von Methan" ein Gasgemisch, insbesondere das nachfolgend erwähnte erste Gasgemisch, gebildet wird, soll hierunter verstanden werden, dass das entsprechende Gasgemisch zumindest einige durch oxidative Kopplung von Methan gebildete Komponenten umfasst. Wie zuvor erläutert, können entsprechende Verfahren bzw. Prozesse auch derart ausgestaltet sein, dass einem katalytischen Schritt bzw. einer katalytischen Reaktionszone ein nichtkatalytischer Schritt bzw. eine nichtkatalytische Reaktionszone in einem entsprechenden Reaktor nachgeschaltet ist. Auch in diesem Fall ist ein entsprechendes Gasgemisch jedoch "unter Einsatz eines Verfahrens zur oxidativen Kopplung von Methan" gebildet. Bei der Bildung sind in diesem Fall lediglich zusätzlich weitere Verfahren bzw. Reaktionen beteiligt.

Die vorliegende Erfindung kann grundsätzlich unter Verwendung unterschiedlichster Reaktoranordnungen realisiert werden. Beispielsweise können parallel oder seriell angeordnete Reaktoren beliebiger Art eingesetzt werden. Insbesondere können rein katalytische und rein nichtkatalytische Reaktoren parallel oder seriell miteinander verbunden sein, es ist jedoch auch möglich, einzelne Reaktoren mit katalytischen und nichtkatalytischen Reaktionszonen beliebiger Art auszustatten.

Komponentengemische, insbesondere Gasgemische, können im hier verwendeten Sprachgebrauch reich oder arm an einer oder mehreren Komponenten sein, wobei der Begriff "reich" für einen Gehalt von wenigstens 75%, 80%, 90%, 95% oder 99% und der Begriff "arm" für einen Gehalt von höchstens 25%, 20%, 10%, 5% oder 1% auf molarer, Gewichts- oder Volumenbasis stehen kann. Ein "überwiegend" eine oder mehrere Komponenten enthaltendes Komponentengemisch ist insbesondere reich an dieser bzw. diesen Komponenten im soeben erläuterten Sinn.

Komponentengemische können im hier verwendeten Sprachgebrauch ferner angereichert oder abgereichert an einer oder mehreren Komponenten sein, wobei sich diese Begriffe auf einen entsprechenden Gehalt in einem anderen Komponentengemisch beziehen, unter Verwendung dessen das betrachtete Komponentengemisch gebildet wurde. Das Komponentengemisch ist "angereichert", wenn es zumindest den 1,5-fachen, 2-fachen, 5-fachen, 10-fachen, 100-fachen oder 1.000-fachen Gehalt der bezeichneten Komponente(n) aufweist, und "abgereichert", wenn es höchstens den 0,75-fachen, 0,5-fachen, 0,1-fachen, 0,01-fachen oder 0,001-fachen Gehalt der bezeichneten Komponente(n) aufweist.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, deren Maximal- und Minimalwerte sich um beispielsweise nicht mehr als 1%, 5%, 10%, 20% oder sogar 50% unterscheiden.

Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste ein. Entsprechendes gilt für Temperaturniveaus. Bei den hier in bar bzw. mbar angegebenen Druckniveaus handelt es sich jeweils um Absolutdrücke.

Im Rahmen der vorliegenden Erfindung können Verdichter zum Einsatz kommen, die insbesondere als mehrstufige Maschinen ausgebildet sein können. Der mechanische Aufbau entsprechender mehrstufiger Verdichter ist dem Fachmann grundsätzlich bekannt. Ein entsprechender mehrstufiger Verdichter, beispielsweise ein Turboverdichter oder Kolbenverdichter, bildet dabei eine bauliche Einheit, in der die mehreren Verdichterstufen integriert sind. Bei einem Turboverdichter umfasst eine Verdichterstufe dabei typischerweise ein Turbinenrad bzw. eine entsprechende Anordnung von Turbinenschaufeln. Bei Kolbenverdichtern sind Kolben mit einer gemeinsamen Welle gekoppelt. Sämtliche Verdichterstufen können dabei von einer gemeinsamen Welle angetrieben werden. Es kann jedoch auch vorgesehen sein, die Verdichterstufen einzeln oder gruppenweise mit unterschiedlichen Wellen anzutreiben, wobei die Wellen auch über Getriebe miteinander verbunden sein können. Als Antrieb für einen entsprechenden Verdichter werden beispielsweise Dampfturbinen oder Elektromotoren eingesetzt.

### Vorteile der Erfindung

Die vorliegende Erfindung geht von einem Prozess zur Herstellung von Ethylen aus, bei dem unter Einsatz eines Verfahrens zur oxidativen Kopplung von Methan ein erstes Gasgemisch gebildet wird. Wie bereits erläutert, können bei der Bildung des ersten Gasgemischs insbesondere auch weitere stoffumwandelnde Prozesse bzw. Verfahren beteiligt sein, insbesondere postkatalytische Dampfspaltverfahren und dergleichen. Im Rahmen der vorliegenden Erfindung wird dabei zumindest ein Teil des ersten Gasgemischs zur Bildung eines zweiten Gasgemischs verwendet. Ist hier davon die Rede, dass "ein Teil" des ersten Gasgemischs zur Bildung eines zweiten Gasgemischs verwendet wird, kann dies sowohl umfassen, dass nur ein mengenmäßiger, jedoch in seiner stofflichen Zusammensetzung unveränderter Anteil des ersten Gasgemischs bei der Bildung des zweiten Gasgemischs verwendet wird, jedoch auch, dass in einer ggf. vorgenommenen Aufbereitung Komponenten des ersten Gasgemischs abgetrennt werden und damit nur ein in seiner stofflichen Zusammensetzung veränderter Teil des ersten Gasgemischs in das zweite Gasgemisch überführt wird. Die Bildung des zweiten Gasgemischs kann dabei auch insbesondere umfassen, zumindest das erste Gasgemisch oder einen entsprechenden Anteil hiervon einer Aufbereitung zu unterwerfen, in der beispielsweise Kohlendioxid und bei Bedarf andere Komponenten entfernt werden können. Eine derartige Aufbereitung kann aber auch stromab der Bildung des zweiten Gasgemischs erfolgen, wie auch nachfolgend noch erläutert. Bei der Bildung des zweiten Gasgemischs kann auch eine Verdichtung des ersten und/oder des gebildeten zweiten Gasgemischs umfasst sein, wie ebenfalls nachfolgend noch erläutert.

Im Rahmen der vorliegenden Erfindung wird dann zumindest ein Teil des entsprechend gebildeten zweiten Gasgemischs, bei dessen Bildung im Rahmen der vorliegenden Erfindung auch ein viertes Gasgemisch verwendet wird und bei dessen Bildung zudem optional weitere Gasgemische verwendet werden können, wie unten erläutert, einer Vakuumdruckwechseladsorption unterworfen. Eine Vakuumdruckwechseladsorption unterscheidet sich von einer regulären Druckwechseladsorption, wie oben erwähnt, durch das zur Desorption verwendete Druckniveau, das unterhalb des Atmosphärendrucks liegt. Eine Vakuumdruckwechseladsorption zeichnet sich insbesondere durch die ihre höhere Effizienz gegenüber einer regulären Druckwechseladsorption aus. Generell steigt der apparative Aufwand dabei mit einer zunehmenden Verringerung des Druckniveaus an. Im Rahmen der Druckwechseladsorption werden dabei ein drittes Gasgemisch und ein viertes Gasgemisch gebildet. Zumindest ein Teil des zweiten Gasgemischs wird also zur Bildung des dritten und des vierten Gasgemischs verwendet.

Das zweite Gasgemisch, das der Druckwechseladsorption zumindest zum Teil zugeführt wird, enthält Methan, Ethan und Ethylen, es kann jedoch auch insbesondere Nebenprodukte der oxidativen Kopplung von Methan und inerte Komponenten enthalten. Wird im Zuge der Bildung des zweiten Gasgemischs eine Aufbereitung vorgenommen, können beispielsweise Wasser und/oder Kohlendioxid vollständig oder nahezu vollständig entfernt worden sein. Letztere Komponenten werden dann im Rahmen der Aufbereitung zuvor abgetrennt.

Die Vakuumdruckwechseladsorption bewirkt dabei, dass das dritte Gasgemisch gegenüber dem zweiten Gasgemisch an Methan angereichert und an Ethan und Ethylen abgereichert ist. Insbesondere ist das dritte Gasgemisch auch an leichter als Methan siedenden Komponenten wie Wasserstoff, Stickstoff und Kohlenmonoxid angereichert, sofern diese in dem zweiten Gasgemisch enthalten sind. Das vierte Gasgemisch ist hingegen gegenüber dem zweiten Gasgemisch an Methan abgereichert und an Ethan und Ethylen angereichert. Insbesondere ist das vierte Gasgemisch auch an Kohlenwasserstoffen mit mehr als zwei Kohlenstoffatomen angereichert, sofern diese in dem zweiten Gasgemisch enthalten sind. Entsprechende Verfahren zur Vakuumdruckwechseladsorption sind dem Grunde nach aus dem Stand der Technik bekannt, wie eingangs erläutert.

Erfindungsgemäß ist jedoch nun vorgesehen, dass ein erster Anteil des vierten Gasgemischs nach der Bildung des ersten Gasgemischs mit dem ersten Gasgemisch oder mit dessen zur Bildung des zweiten Gasgemischs verwendetem Teil vereinigt und schließlich zur Bildung des zweiten Gasgemischs verwendet wird. Mit anderen Worten wird unter Verwendung des ersten Anteils des vierten Gasgemischs ein entsprechender Stoffstrom gebildet, der mit einem unter Verwendung des ersten Gasgemischs gebildeten Stoffstrom vereinigt wird. Es erfolgt also im Rahmen der vorliegenden Erfindung keine Rückspeisung eines entsprechenden Gasgemischs in einen Reaktor, sondern eine Vereinigung mit einem Stoffstrom aus dem Reaktor bzw. aus einer entsprechenden Aufbereitung. Im Rahmen der vorliegenden Erfindung wird ferner ein zweiter Anteil des vierten Gasgemischs anderweitig verwendet, wie nachfolgend im Detail erläutert.

Erfolgt im Rahmen einer besonders vorteilhaften Ausführungsform der vorliegenden Erfindung bei der Bildung des zweiten Gasgemischs eine Verdichtung, kann der erste Anteil des vierten Gasgemischs zusammen mit dem ersten Gasgemisch oder mit dessen zur Bildung des zweiten Gasgemischs verwendetem Anteil, also nach der Vereinigung hiermit, verdichtet werden. Eine besonders bevorzugte Ausgestaltung der vorliegenden Erfindung umfasst also, das zweite Gasgemisch einer Verdichtung zu unterwerfen, so dass auf diese Weise auch der zur Bildung des zweiten Gasgemischs verwendete erste Anteil des vierten Gasgemischs verdichtet werden kann. Alternativ dazu ist es jedoch auch möglich, das zweite Gasgemisch im Wesentlichen auf dem Druckniveau, auf dem das erste Gasgemisch es in der oxidativen Kopplung gebildet wird, der Vakuumdruckwechseladsorption zuzuführen. In diesem Fall wird lediglich der bei der Bildung des zweiten Gasgemischs verwendete erste Anteil des vierten Gasgemischs verdichtet. Gemäß einer weiteren Ausführungsform der vorliegenden Erfindung kann auch, wenngleich eine Verdichtung des ersten Gasgemischs oder dessen zur Bildung des zweiten Gasgemischs verwendetem Anteil erfolgt, der erste Anteil des vierten Gasgemischs erst stromab dieser Verdichtung diesem zugespeist werden. Hierbei kann ein separater Verdichter zur entsprechenden Verdichtung des ersten Anteils des vierten Gasgemischs verwendet werden. In sämtlichen Fällen einer Verdichtung können getrennte oder miteinander verschaltete Maschinen, also getrennte Verdichter oder Verdichterstufen oder mechanisch miteinander gekoppelte Verdichter oder Verdichterstufen, verwendet werden. Auch Mischformen der erläuterten Varianten sind möglich. So kann beispielsweise das erste Gasgemisch oder dessen zur Bildung des zweiten Gasgemischs verwendeter Anteil einer Verdichtung unterworfen, dann mit dem ersten Anteil des vierten Gasgemischs vereinigt, und anschließend gemeinsam mit diesem einer weiteren Verdichtung unterworfen werden. Der erste Anteil des vierten Gasgemischs kann dabei separat verdichtet werden.

Aufgrund der erfindungsgemäß vorgeschlagenen Maßnahmen kann die Effizienz der Vakuumdruckwechseladsorption insgesamt verbessert werden. In einer konventionellen Vakuumdruckwechseladsorption können beispielsweise nicht mehr als 90% des in dem zweiten Gasgemisch enthaltenen Methans in einem einzigen Durchlauf entfernt werden. Für eine weitere Abreicherung bieten sich auf den ersten Blick die Verwendung weiterer Druckwechseladsorptionsstufen an. Allerdings ist es hierzu erforderlich, kostenintensive weitere Verdichterstufen bzw. Verdichter bereitzustellen, die jeweils ein Vakuumdruckwechseladsorption entnommenes Gasgemisch (hier das vierte Gasgemisch bzw. entsprechende Äquivalente) derart verdichten, dass es in eine anschließende Vakuumdruckwechseladsorption eingespeist werden kann. Hierfür sind völlig separat und zusätzlich bereitzustellende Maschinen und eine Druckwechseladsorptionseinrichtungen erforderlich. Grundsätzlich kann eine Effizienzerhöhung einer Vakuumdruckwechseladsorption auch durch eine Druckabsenkung bewirkt werden. Allerdings steigt der apparative Aufwand mit zunehmend sinkendem Druck an.

Im Gegensatz dazu können im Rahmen der vorliegenden Erfindung einfachere und kostengünstigere Maschinen bzw. Anlagenkomponenten eingesetzt werden. So wird durch die Rückführung eines Teils des vierten Gasgemischs dieses nochmals der Vakuumdruckwechseladsorption unterworfen, so dass ein größerer Anteil des Methans und ggf. leichter als Methan siedende Komponenten abgetrennt werden können. Es ergibt sich auf diese Weise eine günstige Beeinflussung der Konzentration der in dem zweiten Gasgemisch enthaltenen Komponenten. Insbesondere wird der Gehalt an schwerer als Methan siedenden Komponenten in dem zweiten Gasgemisch erhöht, so dass das in geringerem Gehalt enthaltene Methan und ggf. leichtere Komponenten effizienter abtrennen lassen.

Durch die erfindungsgemäß vorgeschlagene Rückführung des ersten Anteils des vierten Gasgemischs ist es möglich, die Vakuumdruckwechseladsorption mit einem moderaten Vakuum durchzuführen und dennoch eine hohe Rückgewinnungsrate für Methan zu erzielen, wie sie alternativ nur durch eine deutlich größere Druckabsenkung und/oder eine Erhöhung der Anzahl an Vakuumdruckwechseladsorptionsstufen zu erzielen wäre. Eine derartige Rückführung ist grundsätzlich kontraintuitiv, da der Vakuumdruckwechseladsorption auf diese Weise bereits ein durch diese abgetrenntes Gasgemisch zugeführt wird, also eine Zumischung eines aufgereinigten Produkts der Trennung zu dem noch nicht getrennten Einsatz erfolgt, was auf den ersten Blick das Ergebnis der zuvor durchgeführten Trennung wieder zunichte macht. Durch die insgesamt erhöhte Rückgewinnungsrate an Methan erhöht sich jedoch die Gesamteffizienz des Verfahrens, obwohl in der Vakuumdruckwechseladsorption aufgrund der Rückführung eine größere Gasmenge bearbeitet werden muss.

Wird in einer Ausgestaltung der vorliegenden Erfindung, wie erwähnt, der erste Anteil des vierten Gasgemischs zusammen mit dem ersten Gasgemisch oder dessen zur Bildung des zweiten Gasgemischs verwendeten Teil der Verdichtung unterworfen, ergeben sich weitere Vorteile. Hierzu kann insbesondere ein bereits vorhandener Verdichter verwendet werden, da sich die zu bearbeitenden Volumenströme, wie unten erläutert, nur geringfügig bzw. in einem durch diesen bewältigbaren Umfang erhöhen.

Insgesamt kann im Rahmen der vorliegenden Erfindung durch den Einsatz der vorgeschlagenen Maßnahmen eine sehr viel bessere Abreicherung eines entsprechenden Gasgemischs an Methan erreicht werden. Diese liegt im Rahmen der vorliegenden Erfindung insbesondere bei ca. 90 bis 95% oder mehr, verglichen mit den 80 bis 89% oder weniger, wie sie in herkömmlichen Verfahren erzielt werden. Wie erwähnt, braucht dabei nur eine vergleichsweise moderate Druckabsenkung durchgeführt werden, was mit vergleichsweise geringem technischem Aufwand möglich ist. Gleichwohl kann aber auch eine stärkere Druckabsenkung erfolgen.

Die im Rahmen der vorliegenden Erfindung vorgeschlagenen Maßnahmen sehen also insbesondere eine Rückspeisung eines entsprechenden Tailgases einer Vakuumdruckwechseladsorption vor, die an eine spezifische Stelle in dem Prozess erfolgt. Durch die Rückführung an diese spezifische Stelle, d.h. nach stromab des zur Bildung des ersten Gasgemischs verwendeten Reaktors und gemäß einer besonders bevorzugten Ausführungsform der Erfindung nach stromauf einer Verdichtung, können die erfindungsgemäßen Vorteile erzielt werden.

Mit besonderem Vorteil wird der zweite Anteil des vierten Gasgemischs einem Trennprozess, der insbesondere einem stoffumwandelnden Verfahren zugeordnet sein kann, unterworfen. Der Trennprozess selbst muss dabei nicht stoffumwandelnd sein, sondern schließt sich insbesondere dem stoffumwandelnden Verfahren an. Insbesondere das in dem vierten Gasgemisch enthaltene Ethan kann auf diese Weise abgetrennt und beispielsweise zu Ethylen umgewandelt werden. Mit besonderem Vorteil wird als das stoffumwandelnde Verfahren dabei ein Dampfspaltverfahren eingesetzt, wie es grundsätzlich aus dem Stand der Technik bekannt ist.

Verfahren und Vorrichtungen zum Dampfspalten und zur Aufbereitung der hierbei gewonnenen Gasgemische sind aus dem Stand der Technik in unterschiedlichen Ausgestaltungen bekannt. Zu weiteren Details sei auf einschlägige Fachliteratur, beispielsweise den Artikel "Ethylene" in Ullmann's Encyclopedia of Industrial Chemistry, Onlineausgabe, 2009, DOI 10.2002/14356007.a10 045.pub3, verwiesen. In Dampfspaltverfahren werden Kohlenwasserstoffgemische mit Kohlenwasserstoffen unterschiedlicher Kettenlänge und Struktur gebildet. Um hieraus die gewünschten Produkte, insbesondere Ethylen, zu gewinnen, müssen diese daher abgetrennt werden. Hierzu sind in der genannten Fachliteratur unterschiedliche Trennsequenzen beschrieben. Typischerweise wird hierbei unter anderem eine Fraktion gebildet, die Kohlenwasserstoffe mit zwei Kohlenstoffatomen, insbesondere Ethan und Ethylen, enthält. Diese wird einer sogenannten C2-Trennung zugeführt, in der eine Ethanfraktion und eine Ethylenfraktion gebildet wird. Einer derartigen C2-Trennung oder einem Trennschritt stromauf hiervon wird, ggf. nach einer vorigen Aufbereitung, vorteilhafterweise auch der nicht bei der Bildung des zweiten Gasgemischs verwendete Anteil des vierten Gasgemischs zugeführt. Auf diese Weise kann auf separate Trennschritte zur Abtrennung von Ethylen verzichtet werden. Das in der C2-Trennung anfallende Ethan kann in die Dampfspaltung zurückgeführt werden.

Ein weiterer Aspekt der vorliegenden Erfindung ist also die gezielte Kopplung eines Verfahrens zur oxidativen Kopplung von Methan und eines Dampfspaltverfahrens, durch das sich Synergieeffekte der beiden Verfahren nutzen lassen. Insbesondere kann in dem Dampfspaltverfahren das in der oxidativen Kopplung von Methan ebenfalls gebildete Ethan vorteilhaft genutzt werden. Das in der oxidativen Kopplung von Methan gebildete Ethan und das in dem Dampfspaltverfahren nicht umgesetzte Ethan kann dabei in einem gemeinsamen Trennteil abgetrennt werden.

Im Rahmen der vorliegenden Erfindung kann vorteilhafterweise vorgesehen sein, dass der erste Anteil des vierten Gasgemischs nach einer Trennung von dem zweiten Anteil des vierten Gasgemischs, also separat zu dem zweiten Anteil des vierten Gasgemischs, einer Verdichtung unterworfen wird, bevor dieser mit dem ersten Gasgemisch oder mit dessen zur Bildung des zweiten Gasgemischs verwendetem Teil vereinigt wird. Auf diese Weise kann, falls bei der Bildung des zweiten Gasgemischs keine Verdichtung des ersten Gasgemischs oder dessen jeweils verwendeten Anteils erfolgt, eine Anpassung an das Druckniveau des ersten Gasgemischs erfolgen. Wird eine gemeinsame Verdichtung des ersten Gasgemischs und des zweiten Gasgemischs im Zuge der Bildung des zweiten Gasgemischs vorgenommen, kann insbesondere der Druck dieses ersten Anteils des vierten Gasgemischs an den Saugdruck des dabei verwendeten Verdichters angepasst werden. Dies erfolgt vorteilhafterweise durch die Verwendung eines sogenannten Kreislaufverdichters, der spezifisch mit dem ersten Anteil des vierten Gasgemischs beaufschlagt wird. Ein entsprechender Kreislaufverdichter braucht dabei nur die relativ geringen Volumenströme des ersten Anteils des vierten Gasgemischs zu bearbeiten, so dass deutlich kleinere Maschinen erforderlich sind, als sie bei der Verwendung mehrerer Vakuumdruckwechseladsorptionsstufen erforderlich wären.

In einer Ausgestaltung der Erfindung kann vorgesehen sein, dass der erste Anteil des vierten Gasgemischs zusammen mit dem zweiten Anteil des vierten Gasgemischs einer weiteren Verdichtung unterworfen wird, bevor der erste Anteil und der zweite Anteil des vierten Gasgemischs voneinander getrennt werden. Hierbei kann insbesondere das gesamte Tailgas, also das vierte Gasgemisch, das der Vakuumdruckwechseladsorption auf dem unteratmosphärischen Druckniveau entnommen wird, auf ein Druckniveau gebracht werden, das einem erforderlichen Druck in dem Trennprozess, der dem stoffumwandelnden Verfahren zugeordnet ist, beispielsweise dem Dampfspaltverfahren, entspricht. Da ein entsprechender Verdichter ohnehin vonnöten ist, kann es ggf. vorteilhaft sein, den ersten und den zweiten Anteil des vierten Gasgemischs zunächst gemeinsam zu verdichten.

In anderen Ausgestaltungen der vorliegenden Erfindung kann jedoch auch vorgesehen sein, den ersten und den zweiten Anteil des vierten Gasgemischs voneinander zu trennen, bevor eine separate Verdichtung vorgenommen wird. Hierbei wird die Trennung des ersten Anteils des vierten Gasgemischs und des zweiten Anteils des vierten Gasgemischs auf einem Druckniveau durchgeführt, auf dem sie der Vakuumdruckwechseladsorption entnommen werden, wobei der zweite Anteil des vierten Gasgemischs nach der Trennung einer weiteren Verdichtung unterworfen wird.

Vorteilhafterweise wird im Rahmen der vorliegenden Erfindung in der Vakuumdruckwechseladsorption ein Desorptionsdruckniveau von 100 bis 900 mbar, insbesondere von 200 bis 600 mbar, weiter insbesondere von 300 bis 600 mbar, verwendet. Auf diese Weise lassen sich die erfindungsgemäßen Vorteile in besonderer Weise erzielen. Insbesondere weil, wie erwähnt, im Rahmen der vorliegenden Erfindung eine Rückführung eines Teils des vierten Gasgemischs in die Vakuumdruckwechseladsorption erfolgt, kann moderates Vakuum verwendet werden. Dieses unterschreitet dabei vorzugsweise 300 oder 200 mbar nicht. Grundsätzlich ist jedoch auch ein geringeres Druckniveau einsetzbar.

Vorteilhafterweise wird dabei das zweite Gasgemisch oder dessen der Vakuumdruckwechseladsorption unterworfene Teil der Vakuumdruckwechseladsorption auf einem Druckniveau von 5 bis 30 bar, insbesondere von 8 bis 25 bar, weiter insbesondere 13 bis 17 bar, zugeführt. Die Verdichtung erfolgt daher auf ein entsprechendes Druckniveau, falls dieses noch nicht vorliegt. Wie erwähnt, kann eine Verdichtung ggf. auch unterbleiben, wenn das erste Gasgemisch bereits auf einem geeigneten Druckniveau bereitgestellt wird, also den oder die für die oxidative Kopplung von Methan verwendeten Reaktoren auf einem entsprechenden Druckniveau verlässt. In diesem Fall wird nur der rückgeführte erste Anteil des vierten Gasgemischs mittels eines Kreislaufverdichters oder eines anderen Verdichters auf ein entsprechendes Druckniveau gebracht.

Im Rahmen der vorliegenden Erfindung umfasst vorteilhafterweise der erste Anteil des vierten Gasgemischs 10 bis 80%, insbesondere 20 - 65%, und der zweite Anteil des vierten Gasgemischs den verbleibenden Rest des vierten Gasgemischs. Ein derartiger Recyclestrom reicht zur Erhöhung der Effizienz der Vakuumdruckwechseladsorption in dem zuvor erläuterten Umfang aus, es sind jedoch lediglich Verdichter mit vergleichsweise geringer Dimensionierung erforderlich.

Insbesondere können im Rahmen der vorliegenden Erfindung ein oder mehrere mehrstufige Verdichter verwendet werden. Dabei können sämtliche zuvor erläuterten Verdichtungen bzw. Verdichtungsschritte unter Verwendung von Verdichtungsstufen eines oder mehrerer gemeinsamer Verdichter durchgeführt werden. Beispielsweise kann der erste Anteil des vierten Gasgemischs dabei vollständig durch einen mehrstufigen Verdichter geführt werden, wohingegen der zweite Anteil des vierten Gasgemischs diesem mehrstufigen Verdichter auf einem geringeren Druckniveau entnommen wird. Entsprechendes gilt auch für den zur Bereitstellung des zweiten Gasgemischs verwendeten Verdichter, der ebenfalls mehrstufig ausgebildet sein kann, wobei das erste Gasgemisch oder dessen zur Bildung des zweiten Gasgemischs verwendeter Teil dem Verdichter auf einer Zwischenstufe, der erste Anteil des vierten Gasgemischs diesem Verdichter hingegen voll saugseitig zugeführt wird. In diesem Fall kann auf einen Recycleverdichter verzichtet werden. Sämtliche Verdichterstufen können, wie erwähnt, in einem einzigen Verdichter integriert sein.

Vorteilhafterweise umfasst die Aufbereitung des ersten Gasgemischs oder dessen zur Bildung des zweiten Gasgemischs verwendeten Teils zumindest eine Entfernung von Wasser und Kohlendioxid. Hierfür sind bekannte Verfahrensschritte verfügbar, die eine besonders zuverlässige Entfernung entsprechender Komponenten ermöglichen. Die Aufbereitung kann dabei, wie mehrfach erwähnt, stromauf oder stromab der Vakuumdruckwechseladsorption erfolgen.

Das im Rahmen der vorliegenden Erfindung gebildete zweite Gasgemisch enthält insbesondere 3 bis 20 Molprozent, insbesondere 3 bis 17 Molprozent, an Kohlenwasserstoffen mit zwei und ggf. mehr Kohlenstoffatomen. Das im Rahmen der vorliegenden Erfindung gebildete dritte Gasgemisch enthält insbesondere 0,01 bis 2,5 Molprozent an Kohlenwasserstoffen mit zwei und ggf. mehr Kohlenstoffatomen. Das im Rahmen der vorliegenden Erfindung gebildete vierte Gasgemisch enthält insbesondere 20 bis 80 Molprozent an Kohlenwasserstoffen mit zwei und ggf. mehr Kohlenstoffatomen. Die Angaben beziehen sich dabei, falls die jeweilige Gasgemische Kohlendioxid enthalten, auf den kohlendioxidfreien Anteil. Wie erwähnt, können die angegebenen Gasgemische dann Kohlendioxid enthalten, wenn keine oder nur eine unvollständige Abreicherung stromauf der Vakuumdruckwechseladsorption vorgenommen wird. Hingegen sind die angegebenen Gasgemische arm an oder frei von Kohlendioxid, wenn sie einer entsprechenden Aufbereitung unterworfen wurden. Es versteht sich, dass der Gehalt an Kohlenwasserstoffen mit zwei und ggf. mehr Kohlenstoffatomen in dem dritten Gasgemisch geringer und in dem vierten Gasgemisch höher als in dem zweiten Gasgemisch ist, selbst wenn sich die angegebenen Wertebereiche berühren oder überschneiden. Der verbleibende Anteil der angegebenen Gasgemische ist insbesondere durch Methan und leichter als Methan siedende Verbindungen, falls vorhanden, gebildet. Bei den leichter als Methan siedenden Verbindungen kann es sich insbesondere um Wasserstoff, Stickstoff und/oder Kohlenmonoxid handeln.

Die vorliegende Erfindung betrifft auch eine Anlage zur Herstellung von Ethylen mit den Merkmalen des entsprechenden Patentanspruchs.

Zu weiteren Merkmalen und Vorteilen einer entsprechenden Anlage sei auf die zuvor bezüglich des erfindungsgemäßen Prozesses und seiner Ausgestaltungen erläuterten Aspekte ausdrücklich verwiesen.

Dies gilt auch für die vorteilhafterweise vorgesehene Anlage, die zusätzlich zur Durchführung eines Prozesses eingerichtet ist, wie er zuvor erläutert wurde, und die hierzu eingerichtete Mittel aufweist.

Die Erfindung wird in bevorzugten Ausgestaltungen unter Bezugnahme auf die beigefügten Zeichnungen näher erläutert.

Kurze Beschreibung der Zeichnung
Figur 1 veranschaulicht einen Prozess gemäß einer Ausführungsform der Erfindung in Form eines vereinfachten Prozessflussdiagramms.
Figur 2 veranschaulicht Prozesse gemäß weiterer Ausführungsformen der Erfindung in Form eines vereinfachten Prozessflussdiagramms.

### Ausführliche Beschreibung der Zeichnung

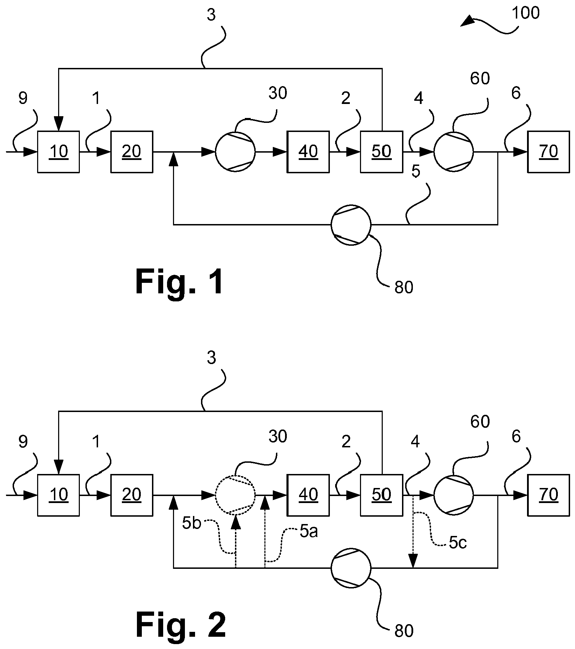

Die Figur 1 veranschaulicht einen Prozess gemäß einer Ausführungsform der Erfindung in Form eines stark vereinfachten Prozessflussdiagramms. Der Prozess ist insgesamt mit 100 bezeichnet.

In dem Prozess 100 wird ein Verfahren 10 zur oxidativen Kopplung von Methan eingesetzt. Unter Einsatz des Verfahrens 10 zur oxidativen Kopplung von Methan wird dabei ein erstes Gasgemisch 1 gebildet. Ein Teil des ersten Gasgemischs 1 wird zur Bildung eines zweiten Gasgemischs 2 verwendet, wie nachfolgend erläutert.

Insbesondere wird das erste Gasgemisch 1 dabei Aufbereitungsschritten 20 und 40 unterworfen, wobei beispielsweise in einem Aufbereitungsschritt 20 ein Quench und eine Entfernung von Wasser erfolgen kann. In einem Aufbereitungsschritt 40 kann insbesondere eine Entfernung von verbleibendem Kohlendioxid durch eine Amin- oder Laugenwäsche erfolgen. Das erste Gasgemisch 1 oder dessen zur Bildung des zweiten Gasgemischs 2 verwendeter Anteil wird dabei insbesondere einer Verdichtung 30 unterworfen, um das zweite Gasgemisch 2 auf einem Druck bereitzustellen, der einem Einspeisedruck einer Vakuumdruckwechseladsorption 50 entspricht.

Der Aufbereitungsschritt 40 kann optional auch stromab der Verdichtung 60 angeordnet oder ein Bestandteil von Prozess 70 sein. Auch kann ggf. auf eine entsprechende Verdichtung 30 verzichtet werden.

Unter Verwendung der Vakuumdruckwechseladsorption 50 werden ein drittes Gasgemisch 3 und ein viertes Gasgemisch 4 gebildet. Das dritte Gasgemisch 3, das gegenüber dem zweiten Gasgemisch 2 an Methan angereichert und an Ethan und Ethylen abgereichert ist, kann insbesondere in das Verfahren 10 zur oxidativen Kopplung von Methan zurückgeführt werden. Letzteres wird ferner mit einem Frischeinsatz 9 gespeist. In dem Verfahren 10 zur oxidativen Kopplung von Methan ebenfalls verwendeter Sauerstoff ist nicht veranschaulicht.

Das in der Vakuumdruckwechseladsorption 50 gebildete vierte Gasgemisch 4 wird der Vakuumdruckwechseladsorption 50 auf einem unteratmosphärischen Druckniveau entnommen und ein in einem Verdichter 60 auf ein Druckniveau gebracht, das eine weitere Verarbeitung, insbesondere in einem Trennprozess, der optional insbesondere einem oder mehreren stoffumwandelnden Prozessen 70 zugeordnet sein kann, ermöglicht. Stromab der Verdichtung 60 wird das vierte Gasgemisch 4 zumindest in einen ersten Anteil 5 und in einen zweiten Anteil 6 aufgeteilt. Der zweite Anteil 5 wird einem Recycleverdichter 80 zugeführt und in diesem auf einen Saugdruck des Verdichters der Verdichtung 30 gebracht. Der erste Anteil 5 wird der Verdichtung 30 zusammen mit dem ersten Gasgemisch oder dessen zur Bildung des zweiten Gasgemischs 2 verwendeten Anteil zugeführt. Ein zweiter Anteil 6 des vierten Gasgemischs 4 wird in den Trennprozess, der dem stoffumwandelnden Verfahren 70 zugeordnet ist, beispielsweise einem Dampfspaltverfahren, überführt.

Figur 2 veranschaulicht Prozesse gemäß weiterer Ausführungsformen der Erfindung in Form eines vereinfachten Prozessflussdiagramms, in dem diese jeweils durch gestrichelte Darstellung veranschaulicht sind. Die gezeigten Alternativen können dabei wahlweise einzeln oder in beliebigen Kombinationen realisiert sein, sofern dies technisch sinnvoll und vorteilhaft ist.

Alternativ zu der Darstellung gemäß Figur 1 kann, wie in Figur 2 in Form eines Stoffstroms 5a gezeigt eine Rückführung des ersten Anteils des vierten Gasgemischs stromab der Verdichtung 30 erfolgen. Wie in Form eines Stoffstroms 5b gezeigt, ist auch eine Rückführung zu einer Zwischenstufe der Verdichtung 5b möglich. Die Verdichtung 30 kann ggf. auch entfallen und es kann lediglich eine Verdichtung in einem Verdichter 60 vorgenommen werden. Wie bereits erwähnt, kann insbesondere in einem derartigen Fall die Aufbereitung 40 auch nach stromab der Vakuumdruckwechseladsorption 50 verlagert werden.

Alternativ zu der Darstellung gemäß Figur 1 kann auch eine Trennung des ersten Anteils des vierten Gasgemischs von dessen zweitem Anteil erfolgen, bevor eine Verdichtung in dem Verdichter 60 vorgenommen wird, wie hier mittels eines gestrichelten Stoffstroms 5c veranschaulicht.

## Patentansprüche

1. Prozess (100) zur Herstellung von Ethylen, bei dem unter Einsatz eines Verfahrens zur oxidativen Kopplung von Methan (10) ein erstes Gasgemisch (1) gebildet wird, bei dem ein Teil des ersten Gasgemischs (1) zur Bildung eines zweiten Gasgemischs (2) verwendet wird, und bei dem zumindest ein Teil des zweiten Gasgemischs (2) einer Vakuumdruckwechseladsorption (50) unterworfen und zur Bildung eines dritten Gasgemischs (3) und eines vierten Gasgemischs (4) verwendet wird, wobei das zweite Gasgemisch (2) Methan, Ethan und Ethylen enthält, wobei das dritte Gasgemisch (3) gegenüber dem zweiten Gasgemisch (5) an Methan angereichert und an Ethan und Ethylen abgereichert ist, und wobei das vierte Gasgemisch (4) gegenüber dem zweiten Gasgemisch (5) an Methan abgereichert und an Ethan und Ethylen angereichert ist, **dadurch gekennzeichnet, dass** ein erster Anteil des vierten Gasgemischs (4) nach der Bildung des ersten Gasgemischs (1) mit dem ersten Gasgemisch (1) oder mit dessen zur Bildung des zweiten Gasgemischs (2) verwendetem Teil vereinigt und zur Bildung des zweiten Gasgemischs (2) verwendet wird, und dass ein zweiter Anteil des vierten Gasgemischs anderweitig verwendet wird.

2. Prozess (100) nach Anspruch 1, bei dem das Bilden des zweiten Gasgemischs eine Verdichtung (30) umfasst, der das erste Gasgemisch oder dessen zur Bildung des zweiten Gasgemischs (2) verwendeter Teil gemeinsam mit dem ersten Anteil des vierten Gasgemischs (4) unterworfen wird.

3. Prozess (100) nach Anspruch 1 oder Anspruch 2, bei dem der zweite Anteil des vierten Gasgemischs (4) einem Trennprozess, der insbesondere einem stoffumwandelnden Verfahren (70) zugeordnet ist, unterworfen wird.

4. Prozess (100) nach Anspruch 3, bei dem das stoffumwandelnde Verfahren (70) ein Dampfspaltverfahren ist, und bei dem der zugeordnete Trennprozess auch zum Trennen eines in dem Dampfspaltverfahren gebildeten Gasgemischs verwendet wird.

5. Prozess (100) nach einem der vorstehenden Ansprüche, bei dem der erste Anteil des vierten Gasgemischs (4) nach einer Trennung von dem zweiten Anteil des vierten Gasgemischs (4) einer Verdichtung (80) unterworfen wird, bevor er mit dem ersten Gasgemisch (1) oder mit dessen zur Bildung des zweiten Gasgemischs (2) verwendetem Teil vereinigt wird.

6. Prozess (100) nach Anspruch 5, bei dem der erste Anteil des vierten Gasgemischs (4) vor der Trennung von dem zweiten Anteil des vierten Gasgemischs (4) zusammen mit dem zweiten Anteil des vierten Gasgemischs (4) einer weiteren Verdichtung (60) unterworfen wird.

7. Prozess (100) nach Anspruch 5, bei dem die Trennung des ersten Anteils des vierten Gasgemischs (4) und des zweiten Anteils des vierten Gasgemischs (4) auf einem Druckniveau erfolgt, auf dem sie der Vakuumdruckwechseladsorption (50) entnommen werden, wobei der zweite Anteil des vierten Gasgemischs nach der Trennung einer weiteren Verdichtung (60) unterworfen wird.

8. Prozess (100) nach einem der vorstehenden Ansprüche, bei dem in der Vakuumdruckwechseladsorption (50) ein Desorptionsdruckniveau von 200 bis 600 mbar, insbesondere von 300 bis 600 mbar, verwendet wird.

9. Prozess (100) nach einem der vorstehenden Ansprüche, bei dem das zweite Gasgemisch oder dessen der Vakuumdruckwechseladsorption (50) unterworfener Teil der Vakuumdruckwechseladsorption (50) auf einem Druckniveau von 8 bis 25 bar zugeführt wird.

10. Prozess (100) nach einem der vorstehenden Ansprüche, bei dem der erste Anteil des vierten Gasgemischs (4) 20 - 65% und der zweite Anteil des vierten Gasgemischs (4) den verbleibenden Rest des vierten Gasgemischs umfasst.

11. Prozess (100) nach einem der vorstehenden Ansprüche, bei dem ein oder mehrere mehrstufige Verdichter verwendet werden, wobei beliebige der angegebenen Verdichtungen (30, 60, 80) gemeinsam oder getrennt voneinander in einem gemeinsamen, in mehreren gemeinsamen und/oder in getrennten Verdichtern durchgeführt werden.

12. Prozess (100) nach einem der vorstehenden Ansprüche, bei dem die Bildung des ersten Gasgemischs eine Aufbereitung des ersten Gasgemischs (1) oder dessen zur Bildung des zweiten Gasgemischs (2) verwendeten Teils unter Entfernung von Wasser und/oder Kohlendioxid umfasst.

13. Anlage zur Herstellung von Ethylen, die dafür eingerichtet ist, unter Einsatz eines Verfahrens zur oxidativen Kopplung von Methan (10) ein erstes Gasgemisch (1) zu bilden, einen Teil des ersten Gasgemischs (1) zur Bildung eines zweiten Gasgemischs (2) zu verwenden, und zumindest einen Teil des zweiten Gasgemischs (2) einer Vakuumdruckwechseladsorption (50) zu unterwerfen und zur Bildung eines dritten Gasgemischs (3) und eines vierten Gasgemischs (4) zu verwenden, wobei das zweite Gasgemisch (2) Methan, Ethan und Ethylen enthält, wobei das dritte Gasgemisch (3) gegenüber dem zweiten Gasgemisch (5) an Methan angereichert und an Ethan und Ethylen abgereichert ist, und wobei das vierte Gasgemisch (4) gegenüber dem zweiten Gasgemisch (5) an Methan abgereichert und an Ethan und Ethylen angereichert ist, **gekennzeichnet durch** Mittel, die dafür eingerichtet sind, einen ersten Anteil des vierten Gasgemischs (4) nach der Bildung des ersten Gasgemischs (1) mit dem ersten Gasgemisch (1) oder mit dessen zur Bildung des zweiten Gasgemischs (2) verwendetem Teil zu vereinigen und zur Bildung des zweiten Gasgemischs (2) zu verwenden, und einen zweiten Anteil des vierten Gasgemischs anderweitig zu verwenden.

14. Anlage nach Anspruch 13, die zur Durchführung eines Prozesses (100) nach einem der Ansprüche 1 bis 12 eingerichtet ist.
